(19) Europäisches Patentamt
European Patent Office
Office européen des brevets

(11) EP 4 641 912 A1

(12) EUROPEAN PATENT APPLICATION
published in accordance with Art. 153(4) EPC

(43) Date of publication:
29.10.2025 Bulletin 2025/44

(51) International Patent Classification (IPC):
$H02N\ 1/04\ ^{(2006.01)}$  $H10N\ 30/87\ ^{(2023.01)}$
$H10N\ 30/05\ ^{(2023.01)}$  $A62B\ 18/10\ ^{(2006.01)}$
$A62B\ 18/02\ ^{(2006.01)}$

(21) Application number: 23907253.1

(52) Cooperative Patent Classification (CPC):
A62B 18/02; A62B 18/10; H02N 1/04; H10N 30/05; H10N 30/87

(22) Date of filing: 22.06.2023

(86) International application number:
PCT/KR2023/008646

(87) International publication number:
WO 2024/135961 (27.06.2024 Gazette 2024/26)

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
KH MA MD TN

(30) Priority: 22.12.2022 KR 20220182007

(71) Applicant: Chung Ang University Industry Academic
Cooperation Foundation
Seoul 06974 (KR)

(72) Inventors:
• LEE, Sangmin
  Gwangmyeong-si Gyeonggi-do 14295 (KR)
• HEO, Deokjae
  Gumi-si Gyeongsangbuk-do 39448 (KR)
• SON, Jinho
  Seoul 01398 (KR)
• KIM, Dongchang
  Anyang-si Gyeonggi-do 14063 (KR)
• SONG, Myeonghwan
  Gwangju 61106 (KR)
• RYU, Hanuk
  Yeoju-si Gyeonggi-do 12631 (KR)
• IN, Subin
  Seoul 06226 (KR)

(74) Representative: ZBM Patents - Zea, Barlocci & Markvardsen
Rambla de Catalunya, 123
08008 Barcelona (ES)

(54) FLUTTER-BASED TRIBOELECTRIC NANOGENERATOR AND OPERATING METHOD THEREFOR

(57) The present disclosure relates to a flutter-based triboelectric nanogenerator and a method of operating the same, and more particularly to a charge accumulation-type, flutter-based triboelectric nanogenerator (CAF-TENG) capable of achieving very high RMS current/average power density and portability, while providing high frequency output and excellent portability by utilizing the fluttering phenomenon of a lightweight sheet and sheet flutter TENG.

[FIG. 11]

EP 4 641 912 A1

**Description**

BACKGROUND

Technical Field

The present disclosure relates to a flutter-based triboelectric nanogenerator and a method of operating the same thereof. More particularly it relates to a charge accumulation-type, flutter-based triboelectric nanogenerator through a discharge gateway and a method of operating the same.

Related Art

Over the past decade, various types of triboelectric nanogenerators (TENG) have been developed depending on target energy harvesting sources such as vibration, airflow, human motion, and precipitation. However, TENGs face challenges in terms of current, frequency and portability, which need to be addressed to enable practical implementation. A major obstacle to application is the extremely low peak current output at the nano/microampere (nA/µA) level, due to the inherent internal impedance and fundamental electrical characteristics of the TENG itself.

Recently, several researchers have attempted to develop high peak current (>milliampere level) TENGs by incorporating additional electrical components, circuits, external grounding, bulky device sizes, etc. However, this results in device packaging issues, which lead to a reduction in portability. In addition, except for ideal cases using automatic input through motors and actuators, it is challenging to sustain a current at high frequencies under regular input conditions while maintaining the mA level. In this regard, there is a need to develop high RMS current and portable TENGs that address these complex issues through an integrated solution.

SUMMARY

Technical Problem

Therefore, the present disclosure is contrived to solve conventional issues as described above. According to an embodiment of the present disclosure, it is an object to provide a charge accumulation-type, flutter-based triboelectric nanogenerator (CAF-TENG) capable of achieving very high RMS current/average power density and portability, while providing high frequency output and excellent portability by utilizing the fluttering phenomenon of a lightweight sheet and sheet flutter TENG.

According to an embodiment of the present disclosure, it is an object to provide a flutter-based triboelectric nanogenerator (CAF-TENG) and a method of operating the same, which is mechanically/electrically designed to generate amplified current output by incorporating a fluttering conductive layer, a charge induction layer and an electrode layer with a discharge gateway structure and which is also capable of generating electrostatic induction output and spark (ESD) as airflow passes through the device due to the charge accumulation and discharge gateway mechanism.

Furthermore, according to an embodiment of the present disclosure, it is an object to provide a flutter-based triboelectric nanogenerator and a method of operating the same, which is structurally optimized through design parameters using an RMS voltage and RMS current database, is applicable in all scenarios where airflow (including wind and respiration) occurs, and exhibits high output performance as demonstrated by multiple LED/commercial lamps, continuous operation of commercial sensor electronic devices and capacitor/battery charging tests.

According to an embodiment of the present disclosure, it is an object to provide a flutter-based triboelectric nanogenerator (CAF-TENG) and a method of operating the same, which can be easily and directly connected (integrated) to commercial mask valves as a practical implementation example. The mask valve-integrated CAF-TENG is capable of functioning as a real-time visual respiration monitoring or safety lighting device in scenarios requiring mask usage, such as construction sites. Furthermore, the energy stored in an energy storage device such as a capacitor during several minutes of natural respiration can be used to supply electrical power to commercial Bluetooth-based electronic devices and transmit wireless signals.

Meanwhile, technical objects to be achieved in the present invention are not limited to the aforementioned technical objects, and other technical objects, which are not mentioned above, will be apparently understood to a person who has ordinary skill in the art from the following description.

Technical Solution

A first aspect of the present disclosure may be achieved by, as a triboelectric nanogenerator, a flutter-based triboelectric nanogenerator including: a first substrate; a first fixed electrode layer coupled to the first substrate; a first charge inducing layer coupled to the first fixed electrode layer; a second charge inducing layer spaced apart from the first charge electrode layer by predetermined distance; a second fixed electrode layer coupled to the second charge inducing layer; and a fluttering conductive layer located in a space between the first charge inducing layer and the second charge inducing layer, wherein it is characterized in that the space between the first charge inducing layer and the second charge inducing layer defines a gas flow space through which gas is introduced and discharged, and the fluttering conductive layer generates output through fluttering behavior induced by the gas flow.

Further, it is characterized in that the first charge inducing layer and the second charge inducing layer are pre-charged with negative charges.

Yet further, it is characterized in that the flutter-based triboelectric nanogenerator further includes a flagpole coupled to the other end of the fluttering conductive layer, wherein the fluttering conductive layer exhibits fluttering behavior with respect to the fluttering conductive layer, and the fluttering conductive layer alternately contacts and separates from the first charge inducing layer and the second charge inducing layer, generating an electrostatic output based on electrostatic induction.

Yet further, it is characterized in that the first fixed electrode layer includes a first discharge gateway, one end of which is exposed to the gas flow space, and the second fixed electrode layer includes a second discharge gateway, one end of which is exposed to the gas flow space.

Yet further, it is characterized in that one end of the fluttering conductive layer alternately contacts and separates from the first discharge gateway and the second discharge gateway, generating a spark output.

Yet further, it is characterized in that the fluttering conductive layer accumulates electrons at one end, + charges are present at the first discharge gateway and the second discharge gateway, such that when contact occurs, the accumulated electrons are simultaneously discharged, generating contact spark output, and when separation occurs, electrons are momentarily re-accumulated, increasing the partial electric filed to generate a separation spark output.

Yet further, it is characterized in that the flagpole is made of a flexible material.

Yet further, it is characterized in that the first and second charge inducing layers are made of PTFE, and the fluttering conductive layer is composed of a Ni/Cu-plated fabric sheet.

A second aspect of the present disclosure may be achieved by, as a method of operating a triboelectric nanogenerator according to any one of claims 1 to claim 8, a method of operating a flutter-based triboelectric nanogenerator including: a first step of introducing a gas into one side of a space between a first charge inducing layer and a second charge inducing layer, and discharging the gas from the other side; a second step of: causing fluttering behavior of a fluttering conductive layer by the flow of the gas; a third step of generating an electrostatic output based on electrostatic induction as the fluttering conductive layer alternately contacts and separates from the first charge including layer and the second charge inducing layer; and a fourth step, performed simultaneously with the third step, of generating a spark output as one end of the fluttering conductive layer alternately contacts and separates from a first discharge gateway and a second discharge gateway.

Further, it is characterized in that, in the fourth step, the fluttering conductive layer accumulates electrons at one end, + charges are present at the first discharge gateway and the second discharge gateway, such that when contact occurs, the accumulated electrons are simultaneously discharged, generating contact spark output, and when separation occurs, electrons are momentarily re-accumulated, increasing the partial electric filed to generate a separation spark output.

A third aspect of the present disclosure may be achieved by, as a mask valve unit coupled to a commercial mask, a flutter-based triboelectric nanogenerator-integrated mask valve unit including: a mask valve having an inlet case into which exhaled air from a user is introduced, a check valve, and a discharge case; and a flutter-based triboelectric nanogenerator according to any one of claim 1 to claim 8, which is coupled to the discharge case of the mask valve and introduces and discharges the exhaled air.

Further, it is characterized in that exhaled air is continuously introduced in one direction by the mask valve, being introduced into one side of a space between a first charge conductive layer and a second conductive layer of the flutter-based triboelectric nanogenerator and discharged from the other side, and the fluttering conductive layer exhibits fluttering behavior, thereby generating electrical output.

Yet further, it is characterized in that the electrical output defines that the fluttering conductive layer alternately contacts and separates from the first charge including layer and the second charge inducing layer, generating an electrostatic output based on electrostatic induction, and simultaneously one end of the fluttering conductive layer alternately contacts and separates from a first discharge gateway and a second discharge gateway, generating a spark output.

Advantageous Effects

According to a charge accumulation-type, flutter-based triboelectric nanogenerator (CAF-TENG) in accordance with an embodiment of the present disclosure, it is capable of achieving very high RMS current/average power density and portability, while providing high frequency output and excellent portability by utilizing the fluttering phenomenon of a lightweight sheet and sheet flutter TENG.

According to a flutter-based triboelectric nanogenerator (CAF-TENG) and a method of operating the same in accordance with an embodiment of the present disclosure, it is mechanically/electrically designed to generate amplified current output by incorporating a fluttering conductive layer, a charge induction layer and an electrode layer with a discharge gateway structure, thereby generating electrostatic induction output and spark (ESD) as airflow passes through the device due to the charge accumulation and discharge gateway mechanism.

Furthermore, according to a flutter-based triboelectric nanogenerator (CAF-TENG) and a method of operating the same in accordance with an embodiment of the present disclosure, it is structurally optimized through design parameters using an RMS voltage and RMS current database, thereby being applicable in all scenarios where airflow (including wind and respiration) occurs, and exhibiting high output performance as demonstrated by multiple LED/commercial lamps, continuous operation of commercial sensor electronic devices and capacitor/battery charging tests.

According to a flutter-based triboelectric nanogenerator (CAF-TENG) and a method of operating the same in accordance with an embodiment of the present disclosure, it is capable of being easily and directly connected (integrated) to commercial mask valves as a practical implementation example. The mask valve-integrated CAF-TENG is capable of functioning as a real-time visual respiration monitoring or safety lighting device in scenarios requiring mask usage, such as construction sites. Furthermore, the energy stored in an energy storage device such as a capacitor during several minutes of natural respiration is capable of being used to supply electrical power to commercial Bluetooth-based electronic devices and transmit wireless signals.

Meanwhile, advantageous effects to be obtained in the present disclosure are not limited to the aforementioned effects, and other effects, which are not mentioned above, will be apparently understood to a person having ordinary skill in the art from the following description.


BRIEF DESCRIPTION OF THE DRAWINGS

The accompanying drawings of this specification exemplify a preferred embodiment of the present disclosure, the spirit of the present disclosure will be more clearly understood from the following detailed description taken in conjunction with the accompanying drawings, and thus it will be understood that the present disclosure is not limited to only contents illustrated in the accompanying drawings.

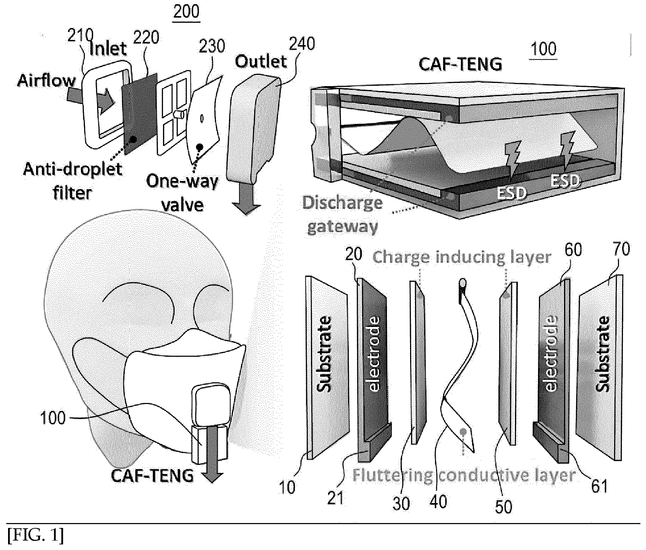

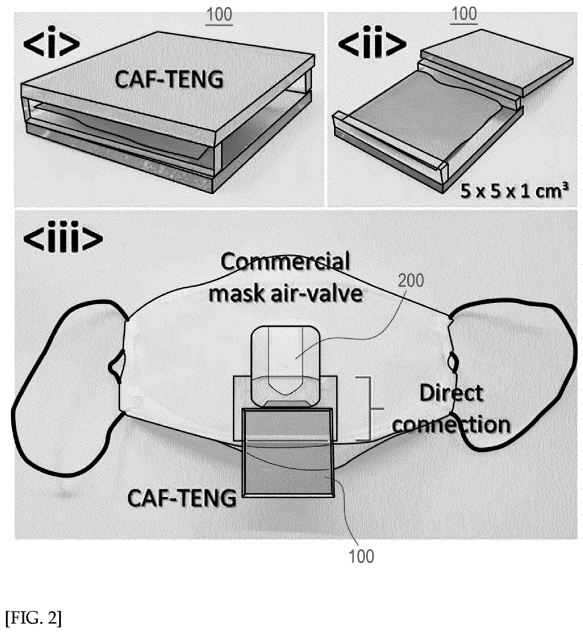

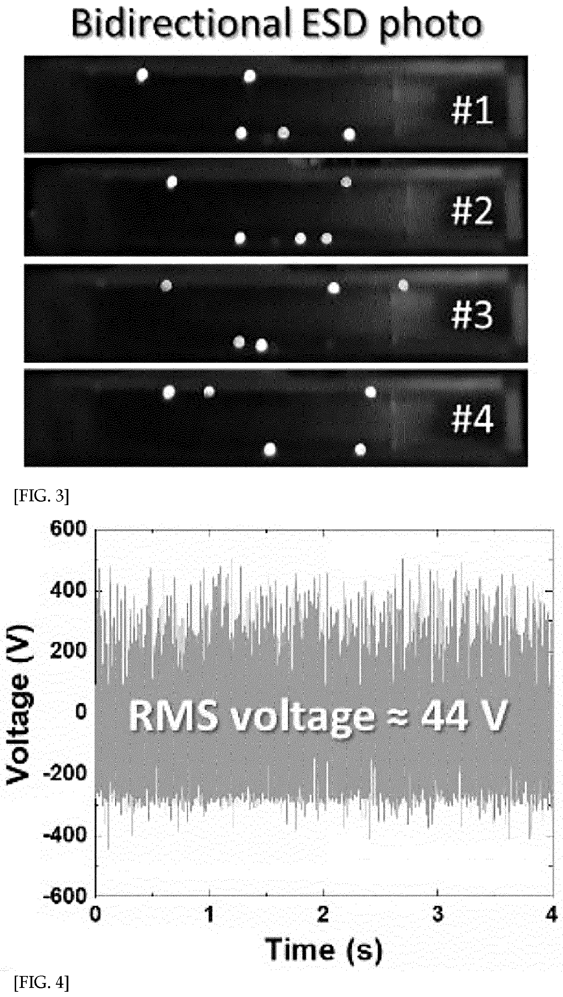

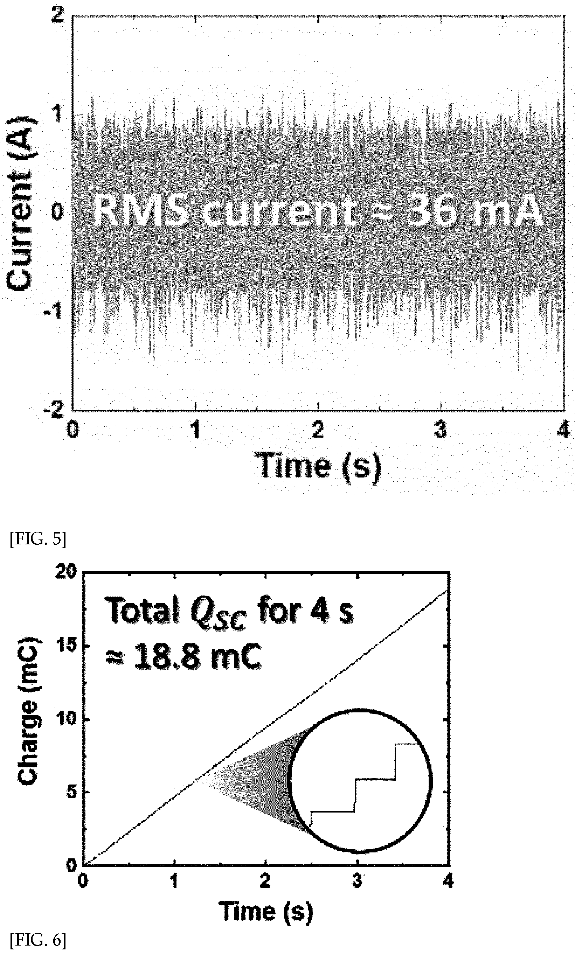

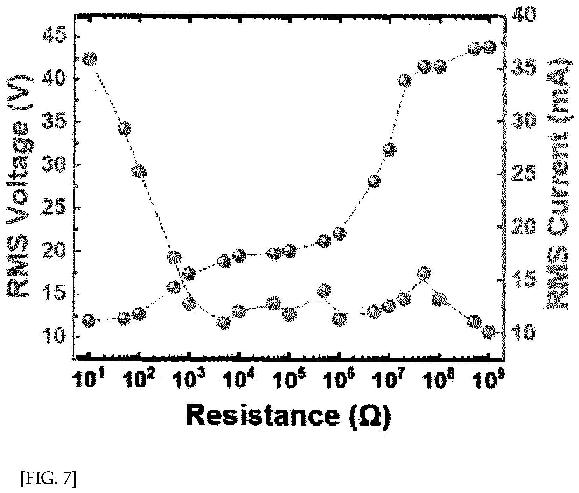

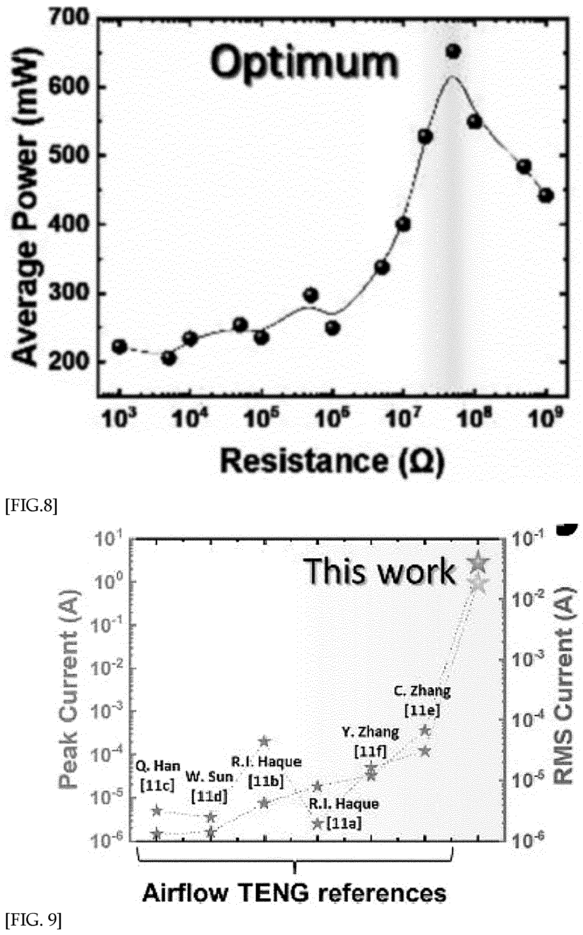

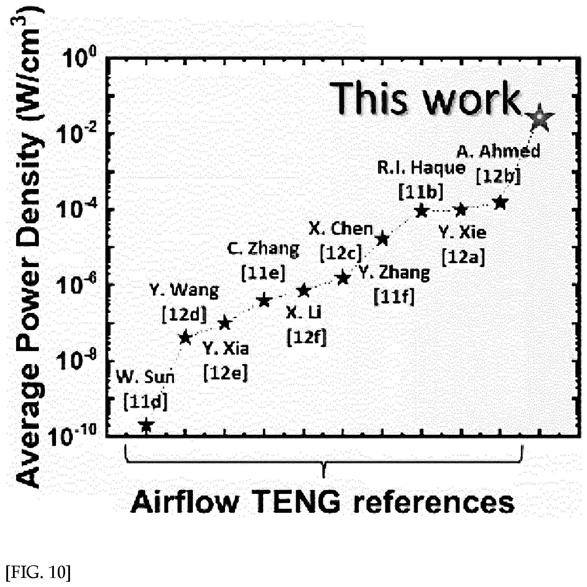

FIGS. 1 to 10 illustrate the concept, structure, and performance of a CAF-TENG according to an embodiment of the present invention. FIG. 1 is a schematic view of a mask valve-integrated CAF-TENG. FIG. 2 is a photograph of the CAF-TENG and a commercial mask air valve integrated design protype. FIG. 3 are bidirectional ESD screenshot images # 1 to #4. FIG. 4 shows the voltage output over 4 seconds. FIG. 5 shows the current output over 4 seconds. FIG. 6 shows the transferred charge output over 4 seconds. FIG. 7 shows the RMS voltage/RMS current and FIG. 8 shows the average power according to external load resistances. FIG. 9 shows the peak current/RMS current, and FIG. 10 shows a comparison of the average power density between the airflow-based TENG according to the conventional art and the CAF-TENG.

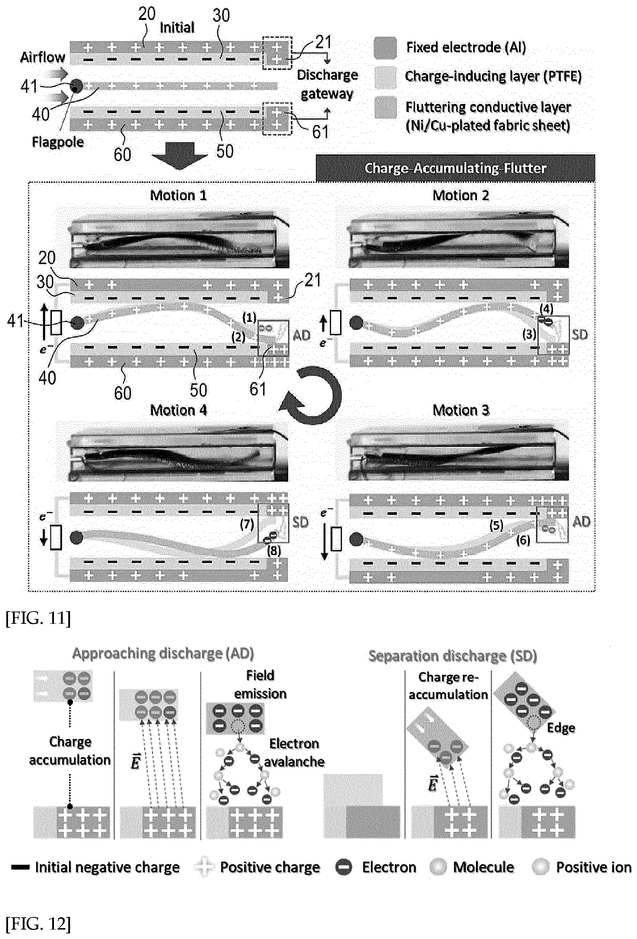

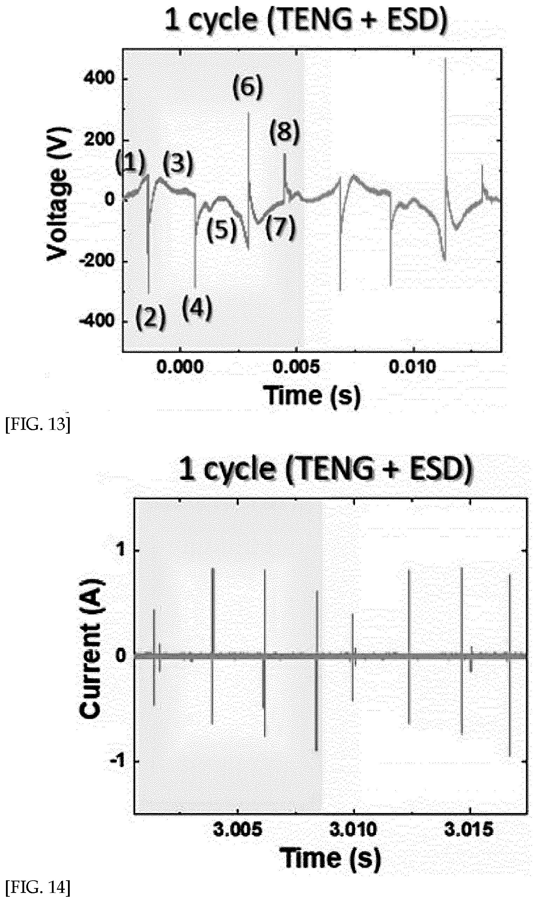

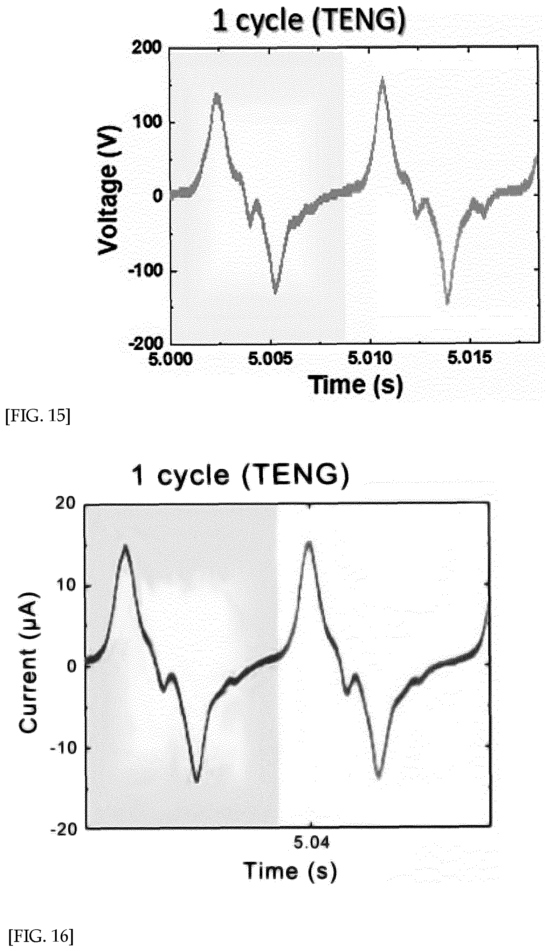

FIG. 11 to FIG. 16 illustrate an operational mechanism of a CAF-TENG according to an embodiment of the present invention. FIG. 11 shows the electrical mechanism of the CAF-TENG generating ultra-high output considering 4 stages of mechanical behavior. FIG. 12 shows two types of ESD outputs: approach discharge (AD) and separation discharge (SD) from the CAF-TENG. FIG. 13 depicts the increased voltage over two cycles including TENG (triboelectric output, electrostatic output) and discharge peaks (spark output). FIG. 14 shows the current output, FIG. 15 shows the increased TENG voltage, and FIG. 16 shows the current output excluding the discharge peaks.

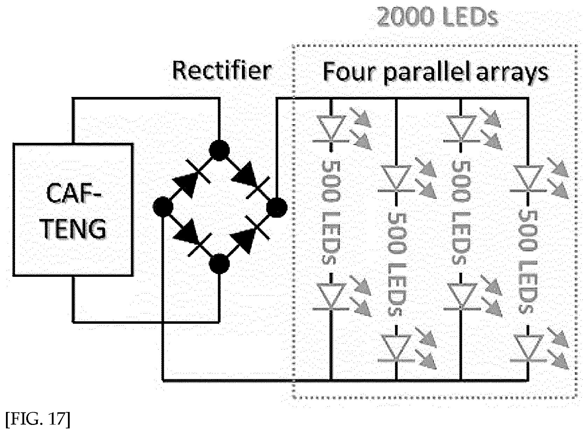

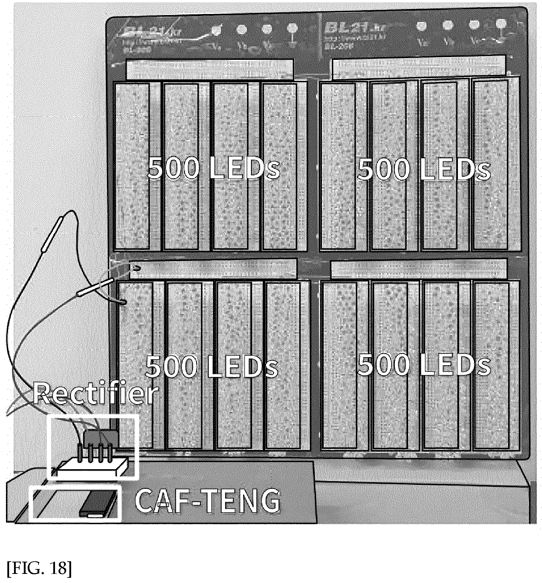

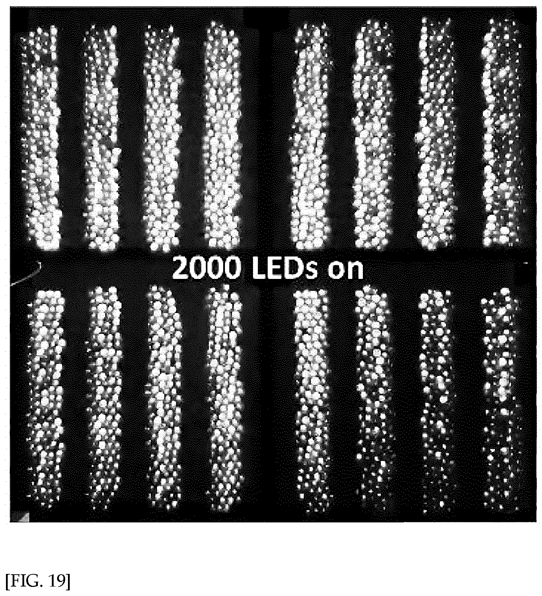

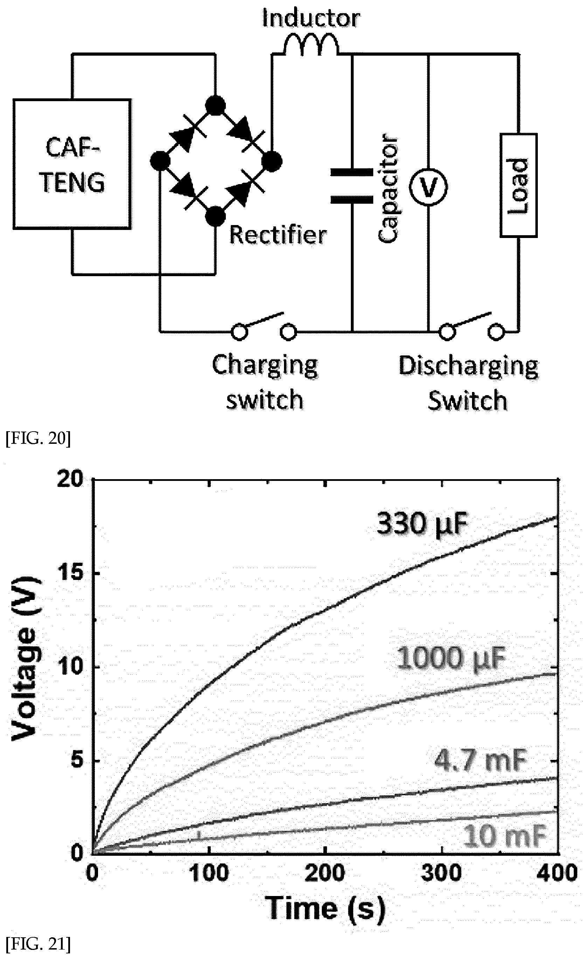

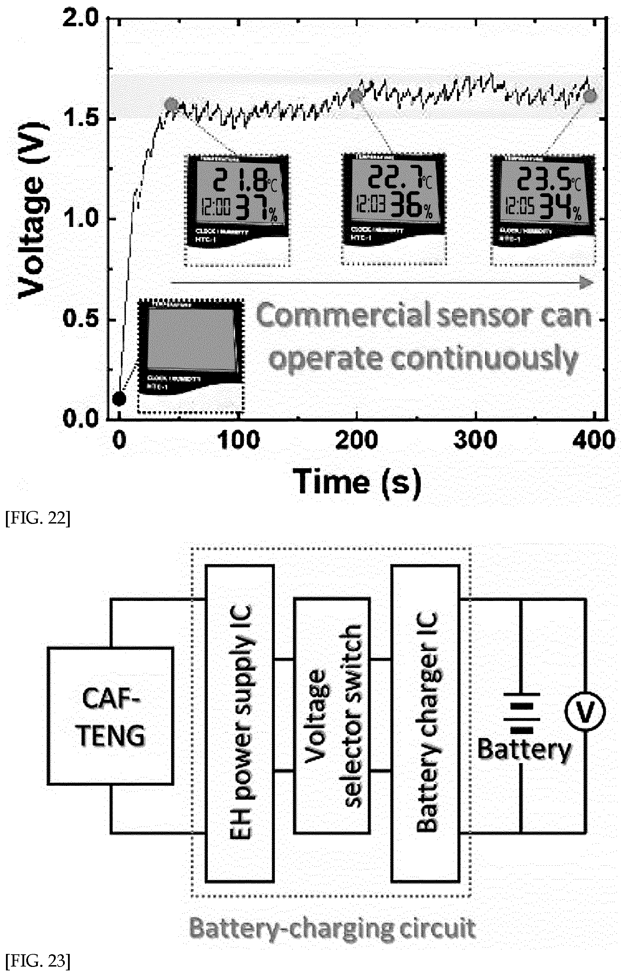

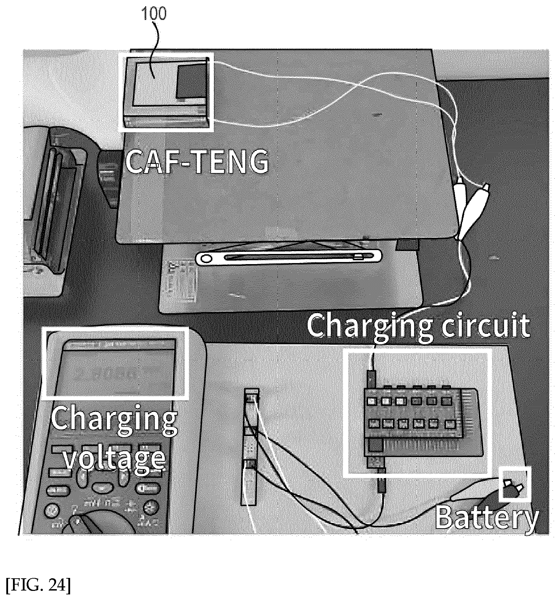

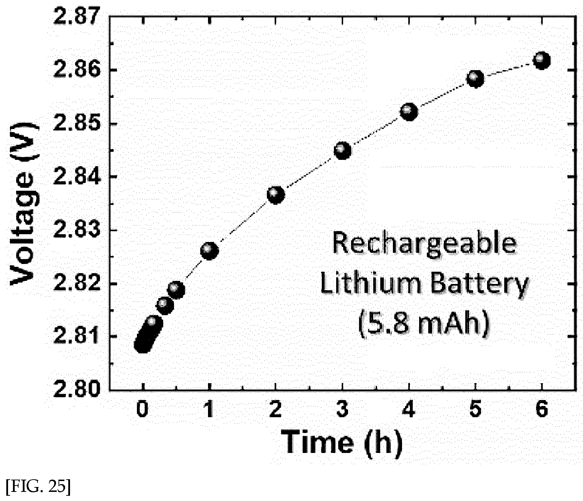

FIG. 17 to FIG. 25 illustrate performance demonstrations of the CAF-TENG under continuous airflow (windy conditions) according to an experimental example of the present disclosure, in accordance with an embodiment of the present disclosure. FIG. 17 shows a rectifier circuit that supplies power to an LED array. FIG. 18 is a setup photograph. FIG. 19 shows 2,000 LEDs operated by the CAF-TENG. FIG. 20 shows a switch circuit configured to charge and discharge a capacitor. FIG. 21 shows charging plots of 330μF, 1000μF, 4.7mF, and 10 F capacitors over 400 seconds. FIG. 22 depicts charging and discharging for continuous operation of a commercial sensor over 400 seconds. FIG. 23 shows a commercial battery charging circuit. FIG. 24 is a setup photograph and FIG. 25 shows a battery charging plot over 6 hours by the CAF-TENG.

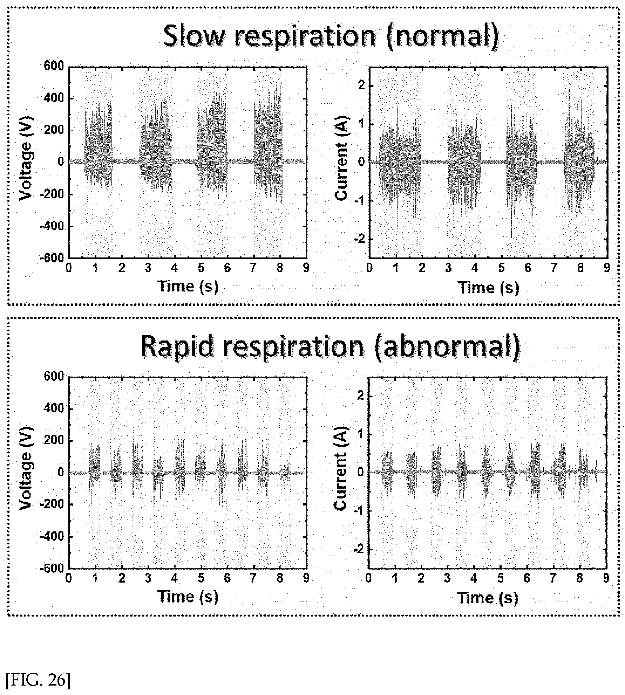

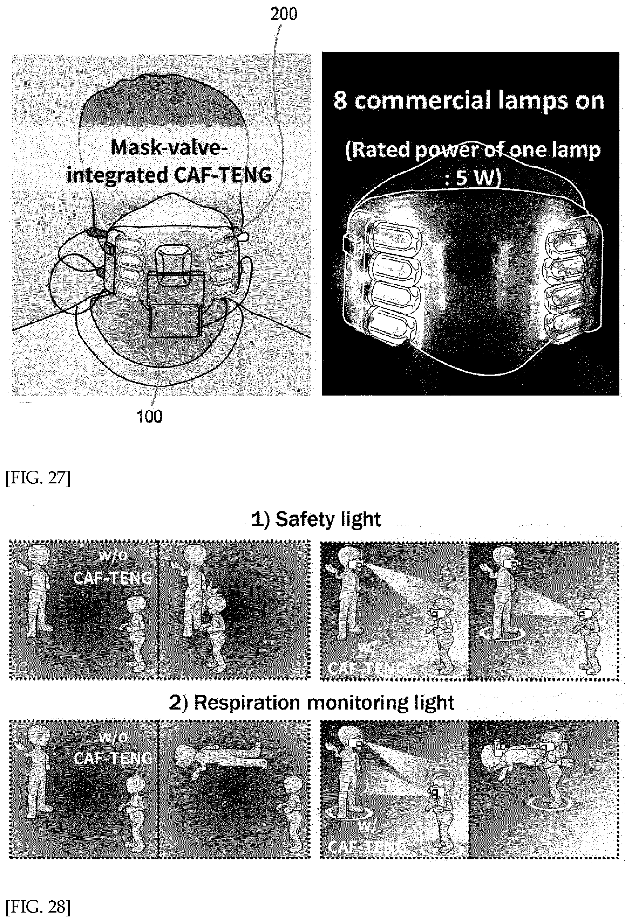

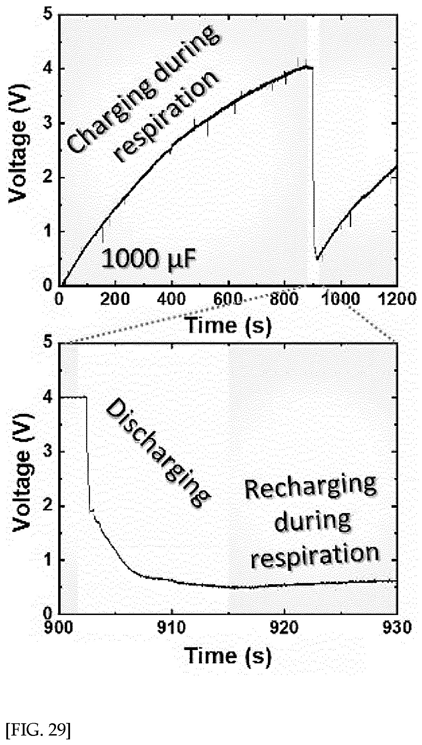

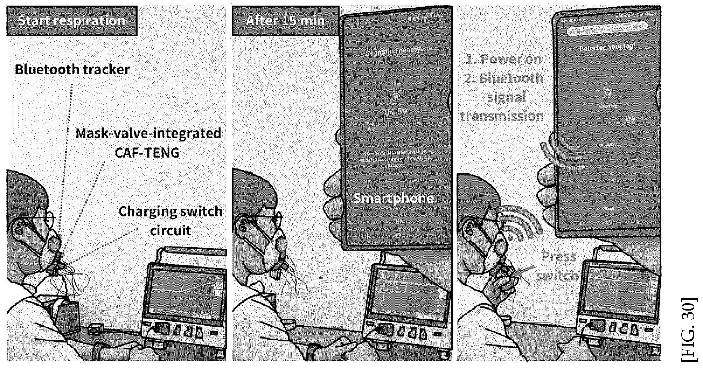

FIG. 26 to FIG. 30 illustrates the mask-integrated CAF-TENG under respiration conditions. FIG. 26 voltage and current output graphs during slow and fast respiration. FIG. 24 shows 8 commercial lamps (5W) being lit by the mask-integrated CAF-TENG during respiration. FIG. 28 shows potential application fields such as safety lights and respiration monitoring lights. FIG. 29 shows a capacitor charging and discharging plot for supplying power to a commercial Bluetooth tracker during respiration. FIG. 30 depicts the operation and signal transmission of the Bluetooth tracker by the mask-integrated CAF-TENG after 15 minutes of respiration.

<Book Antiqua Figure Reference Numbers>

10: first substrate
20: first fixed electrode layer
30: first charge inducing layer
40: fluttering conductive layer
41: flagpole
50: second charge inducing layer
60: second fixed electrode layer
70: second substrate
80: spacer
100: flutter-based triboelectric nanogenerator (CAF-TENG)
200: mask valve

DETAILED DESCRIPTION

Best Mode

Hereinafter, the configuration and functions of a flutter-based triboelectric nanogenerator (hereinafter referred to as CAF-TENG) according to an embodiment of the present disclosure, and the output mechanism (operating method) of the flutter-based triboelectric nanogenerator will be described.

FIG. 1 depicts a schematic view of a mask valve-integrated CAF-TENG. From a practical application perspective, a CAF-TENG 100 may be utilized to harvest various types of airflows. However, the application example of a mask valve 200 was provided as an embodiment to demonstrate the availability of small respiratory input and to highlight miniaturization, portability and everyday mask wearing scenarios. The mask valve 200 is a component installed in commercial safety masks to assist the wearer's easy and comfortable respiration. The commercial mask valve 200 is configured with an inlet case 210, an outlet case 240, a droplet-blocking filter 220 and a check valve 230, and allows only exhaled air to pass through the mask valve due to unidirectional mechanism.

Considering the real structure of the commercial mask valve, the CAF-TENG 100 may be easily integrated into the outlet of the mask valve 200 through a direct sliding connection, thereby enabling immediate use. The exhaled air filtered by the filter 220 of the mask valve 200 continuously generates electricity as it passes through the CAF-TENG 100.

According to an embodiment of the present disclosure, the CAF-TENG 100 is configured with a conductive layer 40 for fluttering charge accumulation, first and second charge inducing layers 30, 50, first and second fixed electrode layers 20, 60, and first and second substrates 10, 70. That is, it has a horizontal symmetry structure. In particular, the first and second fixed electrode layers 20, 60 include discharge gateways 21, 61, thereby significantly amplifying the current output.

The CAF-TENG 100 may simultaneously generate TENG and ESD outputs through the mechanical/electrical interaction between the conductive layer 40, the charge inducing layers 30, 50, the fixed electrode layers 20, 60 and the discharge gateways 21, 61. Therefore, high RMS current/high average power density may be achieved by using small portable devices and small airflow inputs.

Photographs for the actual external and internal structures of the CAF-TENG 100 fabricated according to an embodiment of the present disclosure are shown in FIG. 1-I and FIG. 2-ii, respectively. The external volume of the CAF-TENG 100 including the first and second substrates 10, 70 made of acrylic, is approximately $5 \times 5 \times 1$ cm$^3$. In addition, a photograph for the CAF-TENG 100 directly integrated with the mask valve 200, which is an actual commercial mask, is shown in FIG. 2-iii.

During the operation of the ACF-TENG 100 according to an embodiment of the present disclosure, continuous ESD output is generated between the discharge gateways 21, 61 and one end of the fluttering conductive layer 40. The ESD of the CAF-TENG 100 occurs sequentially at the upper and lower discharge gateways 21, 61. FIG. 3 is a video screen containing 4 screenshot images #1~#4, each corresponding to an individual frame. FIG. 4 to FIG. 6 show the output voltage, short-circuit current (ISC) and total short-circuit transferred charge (QSC) of the CAF_TENG 100 at an airflow rate of 1.5mL/s. The maximum peak voltage and ISC output are 508V and 1.6A, respectively. The output waveform of the CAF_TENG 100 includes both the 120Hz TENG peak and the 480Hx ESD peak, achieving a 44V RMS voltage and a 36mA RMS current. The calculated RMS voltage and current outputs may be derived from the following equations.

[Equation 1]

$$RMS\ voltage\ =\ \sqrt{\frac{\int V^2(t)dt}{T}}\ -\ \sqrt{\frac{\int V^2(t,noise)dt}{T}}$$

[Equation 2]

$$RMS\ current\ =\ \sqrt{\frac{\int I^2(t)dt}{T}}\ -\ \sqrt{\frac{\int I^2(t,noise)dt}{T}}$$

Wherein, V(t) and I(t) represent the raw peak voltage and current outputs during the operation of the CAF-TENG 100, respectively. V(t,noise) and I(t,noise) represent the raw voltage and current measured under ambient electrical noise, respectively. T represents the total measurement period.

The voltage and current outputs of the CAF-TENG 100 according to an embodiment of the present disclosure may vary depending on the external load resistance. As shown in FIG. 7, as the external load resistance increases, the RMS voltage increases, while the RMS current decreases. Furthermore, the average power plot may be obtained by multiplying the RMS voltage and TMS current at each external load resistance. The CAF-TENG 100 generates a maximum average power of 652.3 mW at an optimal load resistance of 50 MΩ (FIG. 8). This electrical output performance of the CAF-TENG 100 represents a dramatically increased level compared to conventional TENGs. In terms of both peak and RMS values, the current output performance of the CEF-TENG 100 is more than 103 times higher than that of previously reported and publicly disclosed airflow based TENG FIG. 9). The average power density is calculated as the average power per device volume. It can be seen that the CAF-TENG 100 generates an average power density of 26mW/cm3, which is more than 102 times higher than that of various conventional airflow based TENGs [11b, 11d-f, 12] (FIG. 10).

Description of Embodiments

Hereinafter, the operational mechanism of a CAF-TENG according to an embodiment of the present disclosure will be described.

As shown in FIG. 11, the CAF-TENG 100 is configured with fixed electrode layers 20, 60 having discharge gateway structures (A1) 21, 61, charge inducing layers (PTFE) 30, 50, a fluttering conductive layer (Ni/Cu plated fabric sheet) 40, a flagpole (PVC) 41 connected to one end o f the fluttering conductive layer 40, and substrates (PMMA) 10, 70. The flagpole 41 is configured to be installed at one end of a fluttering conductive layer 40, and is supported by a small slot of a spacer 80, which spaces substrates 10, 70 apart from each other.

In the initial stage, it is assumed that the charge inducing layers 30, 50 have negative surface charges, and the fixed electrode layers 20, 60 and the fluttering conductive layer 40 are positively charged. When air exceeding a critical flow rate passes through the interior of the CAF-TENG 100, the fluttering conductive layer 40 begins to oscillate and flutter periodically, forming harmonic waves according to the Euler-Bernoulli beam equation and the Navier-Stokes equation.

Then, the central portion of the flexible flagpole 41 also oscillates due to the mechanical force and moment generated by the fluttering of the fluttering conductive layer 40, thereby amplifying the oscillatory motion of the fluttering conductive layer 40. As shown in FIG. 11, the mechanical behavior of the fluttering conductive layer 40 may be categorized into 4 stages, each occurring at time intervals of 2.5 ms.

In Motion 1, the fluttering conductive layer 10 comes into substantial contact with the first charge inducing layer 30 for the first time. However, when its end is suspended in the air ((1) of Motion 1), free electrons flow from the discharge gateway 61 of the first fixed electrode layer 20 to the first fixed electrode layer 20 The negative charges in the charge inducing layers 30, 50 come into equilibrium with the fluttering conductive layer 40 at the contact area, and the electrons in the fluttering conductive layer 40 accumulate toward its end. Subsequently, the end of the fluttering conductive layer 40 approaches the discharge gateway 61 of the second fixed electrode layer 60, the gap between the end and the discharge gateway 61 decreases to the micro scale ((2) of Motion 1).

According to Paschen's law, as the local electric field strength in the micro-scale gap exceeds the breakdown strength of air, a portion of the electrons accumulated at the end of the fluttering conductive layer 40 are emitted through the field emission phenomenon, and an electron avalanche then occurs. The emitted electrons (seed electrons) are accelerated by the strong local electric field, colliding with air molecules and resulting in ionization. The electrons generated by ionization then collide with other air molecules, resulting in a chain reaction. As a result, a conductive path is formed, and an approach discharge (AD) is observed.

In the next Motion 2, the fluttering conductive layer 40 continues to maintain contact with the first charge inducing layer 20, but the contact area decreases (3) and the flow of free electrons from the lower fixed electrode layer 60 to the upper fixed electrode layer 20 decreases. Immediately after the end of the fluttering conductive layer (40) is discharged from the discharge gateway (61) of the second fixed electrode layer (60), the electrons of the fluttering conductive layer 40 rapidly move toward the end and are re-accumulated by the negative charges in the charge inducing layers 30, 50. When assuming that the fluttering conductive layer 40 is still affected by the same contact area as that of the charge inducing layers 30, 50, as the end of the fluttering conductive layer 40 moves away from the discharge gateway 61 of the second fixed electrode layer 60, the electric field strength between the ends momentarily increases. Then, when the electric field exceeds the breakdown strength of air withing the micro-scale gap, field emission and electron avalanche occur. This is separation discharge (SD), which is a different discharge case from AD.

In Motion 3 and Motion 4, a similar electric generation process is repeated. In Motion 3, the fluttering conductive layer 40 comes into contact with the second charge inducing layer 50, but the end of the fluttering conductive layer 40 is suspended in the air (5). At that time, free electrons flow from the discharge gateway 21 of the first fixed electrode layer 20 toward the second fixed electrode layer 60, and the electrons in the fluttering conductive layer 40 are concentrated at the end. While the end of the fluttering conductive layer 40 approaches the discharge gateway 21 of the first fixed electrode layer 20, the local electric field strength between them rapidly increases, and the electrons accumulated at the end are emitted to the discharge gateway through the approach discharge (AD).

In Motion 4, while the fluttering conductive layer 40 continues to contact with the second charge inducing layer 50, the contact area decreases 7, resulting in a reduced flow of free electrons from the first fixed electrode layer 20 to the second fixed electrode layer 60. Then, the end of the fluttering conductive layer 40 begins to separate from the discharge gateway 21 of the first fixed electrode layer 20, the electrons in the fluttering conductive layer are accumulated at the end. Even if a slight gap distance occurs between them, a strong electric field is momentarily generated and increases dramatically.

When the electric field exceeds the breakdown strength of air, electron avalanche and SD occur similarly to Motion 2. FIG. 2b shows a detailed schematic view of the above-mentioned AD and SD. The key point in the generation of AD and SD is the induction of localized electron accumulation in the fluttering conductive layer 40 under the strong electric field of the charge inducing layers 30, 50, which come into contact with the discharge gateways 21, 61.

According to the electric mechanism as illustrated in FIG. 11 and FIG. 12, the CAF-TENG 100 generates and sequentially discharges TENG output during one cycle (Motion 1 ~ 4). The enlarged voltage and current outputs of the CAF-TENG 100 over two cycles are shown in FIG, 13 and FIG. 14.

In FIG, 13, each sub-action of (1)-(3)-(5)-(7) and (2)-(4)-(6)-(8) corresponds to one cycle of TENG and discharge output. Meanwhile, in the current output waveform shown in Fig. 14, only the discharge peaks are observed due to the small scale of the TENG current. The maximum discharge voltage and ISC are 472 V and 0.94 A, respectively.

FIG. 15 and FIG. 16 show the enlarged TENG voltage and current outputs excluding the discharge peaks with a maximum voltage of 160 V and a current of 15.4 $\mu$A. Therefore, the CAF-TENG 100 may obtain both high peak and RMS outputs.

Hereinafter, the performance experimental data for the CAF-TENG 100 under continuous airflow (windy) conditions according to an embodiment of the present disclosure will be described.

The CAF-TENG 100 according to an embodiment of the present disclosure has the potential to function as a small portable wind energy harvester under a daily continuous airflow condition. High current output and high average power density performance may be utilized to implement instantaneous power for electrical elements, components, and devices. As one application example, the CAF-TENG 100 may continuously supply power to a commercial LED array of 2000 LEDs through a simple rectifier circuit.

FIG. 17 shows a detailed circuit consisting of the CAF-TENG 100, a wave bridge rectifier, and 2000 LEDs (4 parallel connections of 500 LEDs each connected in series). FIG. 18 an actual experimental setup for supplying power to the 2000 LEDs. As shown in FIG. 18, the 2000 LED array may be illuminated only by the CAF-TENG 100. Considering these results, when the CAF-TENG 100 is installed in interior and exterior spaces exposed to high wind, it may function as a safety/indicator light securing high visibility for pedestrians or drivers.

Furthermore, the CAF-TENG 100 may charge various commercial energy storage devices such as capacitors and batteries, under high wind conditions. FIG. 20 shows a capacitor charging and load discharging circuit including a wave bridge rectifier, an inductor, a capacitor and a load. In addition, FIG. 21 shows a charging plot for various high-capacity capacitors.

The CAF-TENG 100 successfully charged capacitors of 330 $\mu$F, 1000 $\mu$F, 4.7 mF and 10 mF at an airflow rate of 1.5mL/s. When continuous airflow is generated, the CAF-TENG 100 charges the capacitors and may supply power to commercial sensors for several seconds. In addition, the commercial sensors may operate continuously without any time limitations. FIG. 22 shows that the CAF-TENG 100, may simultaneously charge and discharge, operating commercial digital temperature and humidity meter/clock sensor continuously, unlike a conventional TENG. In the graph, the charging and discharging rate of the sensor is balanced, maintaining a capacitor voltage of approximately 1.5V, which is the rated voltage of the sensor.

Additionally, the CAF-TENG 100 may charge a rechargeable lithium battery. For safe and effective charging, a commercial battery charging circuit, which includes an EH (Energy Harvesting) power management integrated circuit (IC), a voltage selection switch and a battery charging IC (FIG. 23), was used. When the CAF-TENG 100 generates irregular electricity output, the battery charging circuit temporarily stores the charge inside the capacitor, then immediately transferring it to the battery. This energy transferring process is rapidly repeated due to the high frequency operation of the CAF-TENG 100. The photograph of the battery charging experiment is shown in FIG. 24. The CAF-TENG 100 may charge 5.8 mAh (rated voltage of 3 V). As shown in FIG. 25, the lithium battery increased from 2.808 V to 2.861 V within 6 hours.

Hereinafter, the experimental data for the mask-integrated CAF-TENG under respiration conditions will be described.

The CAF-TENG 100 may operate through a self-powered portable device integrated with a mask valve 200. As mentioned in FIG. 1 and FIG. 2, the CAF-TENG 100 may be designed to be directly integrated with a commercial mask air valve, operating in everyday life under respiration conditions. FIG. 26 shows the voltage and current of the mask valve 200-integrated CAF-TENG 100 under two types of respiration conditions. For slow respiration (normal breathing), the mask valve 200-integrated CAF-TENG 100 generated maximum voltage and current of 508 V and 1.98 A, respectively.

Furthermore, the mask valve 200-integrated CAF-TENG 100 generated a peak voltage and current of 238 V and 1.02 A, respectively, under fast respiration conditions (abnormal breathing). The respiration-based instantaneous high output performance of this mask valve 200-integrated CAF-TENG 100 may be applied to a commercial lamp (rated power consumption of 5 W). FIG. 27 shows a commercial lamp array of 8 lamps, which is continuously illuminated by the mask valve 200-integrated CAF-TENG 100 during respiration.

The instantaneous output generated by respiration is rectified using a wave bridge rectifier. In the present application example, it may be utilized as a real-time safety light and respiration monitoring light. Considering dark/mask wearing scenarios such as in construction sites, exercises, etc., as shown in FIG. 28, the portable lamp light, which is driven by the mask valve 200-integrated CAF-TENG 100, may prevent collision between wearers, workers, pedestrians, and vehicles without any additional devices, and recognize each mask's respiration status.

Since the CAF-TENG 100 exhibits sufficient respiration driven output, it may charge a high-capacity capacitor with just a few minutes of respiration. The stored energy may be used to operate commercial low-power electronic devices. In an experimental example of the present disclosure, as shown in FIG. 29, a 1000 $\mu$F capacitor successfully charges and discharges a commercial Bluetooth tracker to 4 V by the mask valve 200-integrated CAF-TENG 100 during 15 minutes of (slow and fast) mixed respiration, and then the capacitor is stably recharged by respiration. The charging-discharging circuit includes the CAF-TENG 100, a wave bridge rectifier, an inductor, a capacitor and a Bluetooth tracker. While a smartphone (Galaxy Note 20, SAMSUNG Co., South Korea) searched for the location of the tracker in real time using the "SmartThings Fing" service, the tracker was driven by the stored energy, detected by the smartphone via Bluetooth, and wireless data transmission and reception occurred for several seconds (FIG. 30). Therefore, if necessary, all users of the mask valve 200-integrated CAF-TENG 100 may share their locations with each other every few minutes without using any external batteries. These results show that the mask valve 200-integrated CAF_TENG 200 may be directly applied to real-world situations as a respiration driven portable power supply. Additionally, Self-powered IoT sensor network may be also constructed by utilizing self-powered safety lights, respiration monitoring lights, commercial sensor signal lights.

Further, the configuration and method of the embodiments as described above are not restrictively applied to the aforementioned apparatus and method. The whole or part of the respective embodiments may be selectively combined so as to make various modifications of the embodiments.

**Claims**

**1.** As a triboelectric nanogenerator, a flutter-based triboelectric nanogenerator comprising:

a first substrate;
a first fixed electrode layer coupled to the first substrate;
a first charge inducing layer coupled to the first fixed electrode layer;
a second charge inducing layer spaced apart from the first charge electrode layer by predetermined distance;
a second fixed electrode layer coupled to the second charge inducing layer; and
a fluttering conductive layer located in a space between the first charge inducing layer and the second charge inducing layer, wherein
the space between the first charge inducing layer and the second charge inducing layer defines a gas flow space through which gas is introduced and discharged, and the fluttering conductive layer generates output through fluttering behavior induced by the gas flow.

2. The flutter-based triboelectric nanogenerator of claim 1, wherein
the first charge inducing layer and the second charge inducing layer are pre-charged with negative charges.

3. The flutter-based triboelectric nanogenerator of claim 2, further comprising a flagpole coupled to the other end of the fluttering conductive layer; wherein

the fluttering conductive layer exhibits fluttering behavior with respect to the fluttering conductive layer, and
the fluttering conductive layer alternately contacts and separates from the first charge inducing layer and the second charge inducing layer, generating an electrostatic output based on electrostatic induction.

4. The flutter-based triboelectric nanogenerator of claim 3, wherein

the first fixed electrode layer includes a first discharge gateway, one end of which is exposed to the gas flow space, and
the second fixed electrode layer includes a second discharge gateway, one end of which is exposed to the gas flow space.

5. The flutter-based triboelectric nanogenerator of claim 4
one end of the fluttering conductive layer alternately contacts and separates from the first discharge gateway and the second discharge gateway, generating a spark output.

6. The flutter-based triboelectric nanogenerator of claim 5 wherein
the fluttering conductive layer accumulates electrons at one end, + charges are present at the first discharge gateway and the second discharge gateway, such that when contact occurs, the accumulated electrons are simultaneously discharged, generating contact spark output, and when separation occurs, electrons are momentarily re-accumulated, increasing the partial electric filed to generate a separation spark output.

7. The flutter-based triboelectric nanogenerator of claim 3, wherein
the flagpole is made of a flexible material.

8. The flutter-based triboelectric nanogenerator of claim 1
the first and second charge inducing layers are made of PTFE, and the fluttering conductive layer is composed of a Ni/Cu-plated fabric sheet.

9. As a method of operating a triboelectric nanogenerator according to any one of claims 1 to claim 8, a method of operating a flutter-based triboelectric nanogenerator comprising:

a first step of introducing a gas into one side of a space between a first charge inducing layer and a second charge inducing layer, and discharging the gas from the other side;
a second step of: causing fluttering behavior of a fluttering conductive layer by the flow of the gas;
a third step of generating an electrostatic output based on electrostatic induction as the fluttering conductive layer alternately contacts and separates from the first charge including layer and the second charge inducing layer; and
a fourth step, performed simultaneously with the third step, of generating a spark output as one end of the fluttering conductive layer alternately contacts and separates from a first discharge gateway and a second discharge gateway.

10. The method of operating the triboelectric nanogenerator of claim 9, wherein
in the fourth step, the fluttering conductive layer accumulates electrons at one end, + charges are present at the first discharge gateway and the second discharge gateway, such that when contact occurs, the accumulated electrons are simultaneously discharged, generating contact spark output, and when separation occurs, electrons are momentarily re-accumulated, increasing the partial electric filed to generate a separation spark output.

11. As a mask valve unit coupled to a commercial mask, a flutter-based triboelectric nanogenerator-integrated mask valve unit comprising:

a mask valve having an inlet case into which exhaled air from a user is introduced, a check valve, and a discharge case; and
a flutter-based triboelectric nanogenerator according to any one of claim 1 to claim 8, which is coupled to the discharge case of the mask valve and introduces and discharges the exhaled air.

12. The flutter-based triboelectric nanogenerator-integrated mask valve unit of claim 11, wherein exhaled air is continuously introduced in one direction by the mask valve, being introduced into one side of a space between a first charge conductive layer and a second conductive layer of the flutter-based triboelectric nanogenerator and discharged from the other side, and the fluttering conductive layer exhibits fluttering behavior, thereby generating electrical output.

13. The flutter-based triboelectric nanogenerator-integrated mask valve unit of claim 12, wherein

the electrical output defines that
the fluttering conductive layer alternately contacts and separates from the first charge including layer and the second charge inducing layer, generating an electrostatic output based on electrostatic induction, and simultaneously one end of the fluttering conductive layer alternately contacts and separates from a first discharge gateway and a second discharge gateway, generating a spark output.

[FIG. 1]

[FIG. 2]

EP 4 641 912 A1

Bidirectional ESD photo

[FIG. 3]

RMS voltage ≈ 44 V

[FIG. 4]

13

[FIG. 5]

[FIG. 6]

[FIG. 7]

[FIG.8]

[FIG. 9]

[FIG. 10]

[FIG. 11]

[FIG. 12]

[FIG. 13]

[FIG. 14]

[FIG. 15]

[FIG. 16]

[FIG. 17]

[FIG. 18]

2000 LEDs on

[FIG. 19]

[FIG. 20]

[FIG. 21]

[FIG. 22]

[FIG. 23]

[FIG. 24]

[FIG. 25]

[FIG. 26]

[FIG. 27]

1) **Safety light**

2) **Respiration monitoring light**

[FIG. 28]

[FIG. 29]

[FIG. 30]

## INTERNATIONAL SEARCH REPORT

| International application No. |
|---|
| **PCT/KR2023/008646** |

### A. CLASSIFICATION OF SUBJECT MATTER

**H02N 1/04**(2006.01)i; **H10N 30/87**(2023.01)i; **H10N 30/05**(2023.01)i; **A62B 18/10**(2006.01)i; **A62B 18/02**(2006.01)i

According to International Patent Classification (IPC) or to both national classification and IPC

### B. FIELDS SEARCHED

Minimum documentation searched (classification system followed by classification symbols)

H02N 1/04(2006.01); A61B 5/08(2006.01); A61B 5/087(2006.01); A62B 18/04(2006.01); F03D 9/25(2016.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 마찰대전나노발전기(TENG), 기판(substrate), 전극층(electrode layer), 전하유도층(charge-induced layer), 플러터(flutter), 마스크(mask), 밸브(valve)

### C. DOCUMENTS CONSIDERED TO BE RELEVANT

| Category* | Citation of document, with indication, where appropriate, of the relevant passages | Relevant to claim No. |
|---|---|---|
| X | CN 215580953 U (NORTHEAST FORESTRY UNIVERSITY) 18 January 2022 (2022-01-18) See paragraphs [0040]-[0049], claim 2 and figures 1-9. | 1-3,7,8 |
| Y | | 11,12 |
| A | | 4-6,9,10,13 |
| Y | KR 10-2132098 B1 (CHUNG ANG UNIVERSITY INDUSTRY ACADEMIC COOPERATION FOUNDATION) 08 July 2020 (2020-07-08) See paragraphs [0023]-[0026] and figures 1-6. | 11,12 |
| A | CN 114847924 A (HANGZHOU DIANZI UNIVERSITY) 05 August 2022 (2022-08-05) See claims 1-5 and figure 1. | 1-13 |
| A | KR 10-2019-0098481 A (KOREA ELECTRONICS TECHNOLOGY INSTITUTE) 22 August 2019 (2019-08-22) See paragraphs [0083]-[0093] and figure 5. | 1-13 |

☑ Further documents are listed in the continuation of Box C.  ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **22 September 2023** | **25 September 2023** |

| Name and mailing address of the ISA/KR | Authorized officer |
|---|---|
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

| International application No. |
| --- |
| **PCT/KR2023/008646** |

**C.  DOCUMENTS CONSIDERED TO BE RELEVANT**

| Category* | Citation of document, with indication, where appropriate, of the relevant passages | Relevant to claim No. |
| --- | --- | --- |
| A | CN 103780128 A (NATIONAL CENTER FOR NANOSCIENCE AND TECHNOLOGY(NCNST) OF CHINA) 07 May 2014 (2014-05-07)<br>See claim 1 and figures 1-5. | 1-13 |

Form PCT/ISA/210 (second sheet) (July 2022)

**INTERNATIONAL SEARCH REPORT**
Information on patent family members

| International application No. |
| --- |
| **PCT/KR2023/008646** |

| Patent document cited in search report | | | Publication date (day/month/year) | Patent family member(s) | | | Publication date (day/month/year) |
| --- | --- | --- | --- | --- | --- | --- | --- |
| CN | 215580953 | U | 18 January 2022 | None | | | |
| KR | 10-2132098 | B1 | 08 July 2020 | None | | | |
| CN | 114847924 | A | 05 August 2022 | None | | | |
| KR | 10-2019-0098481 | A | 22 August 2019 | WO | 2019-160285 | A1 | 22 August 2019 |
| CN | 103780128 | A | 07 May 2014 | CN | 103780128 | B | 06 April 2016 |
| | | | | WO | 2014-169724 | A1 | 23 October 2014 |

Form PCT/ISA/210 (patent family annex) (July 2022)